(19) Europäisches Patentamt
European Patent Office
Office européen des brevets

(11) Veröffentlichungsnummer: **0 289 935 B1**

(12) # EUROPÄISCHE PATENTSCHRIFT

(45) Veröffentlichungstag der Patentschrift: **24.06.92**

(51) Int. Cl.⁵: **C09K 15/26**

(21) Anmeldenummer: **88106785.4**

(22) Anmeldetag: **28.04.88**

(54) **Stabilisatorgemisch aus organischen Schwefelverbindungen und organischen Stickstoffverbindungen und Verwendung des Stabilisatorgemisches zur Stabilisierung von Oxalkylenglykolalkylethern.**

(30) Priorität: **02.05.87 DE 3714754**

(43) Veröffentlichungstag der Anmeldung:
**09.11.88 Patentblatt 88/45**

(45) Bekanntmachung des Hinweises auf die Patenterteilung:
**24.06.92 Patentblatt 92/26**

(84) Benannte Vertragsstaaten:
**AT BE CH DE FR GB IT LI NL SE**

(56) Entgegenhaltungen:
**DE-A- 3 537 839**
**GB-A- 1 479 708**
**US-A- 4 604 417**
**US-A- 4 717 502**

(73) Patentinhaber: **HOECHST AKTIENGESELL-SCHAFT**
**Postfach 80 03 20**
**W-6230 Frankfurt am Main 80(DE)**

(72) Erfinder: **Stankowiak, Achim, Dr.**
**Ortlehnerstrasse 20**
**W-8269 Burgkirchen(DE)**
Erfinder: **Streitberger, Horst**
**Josef-Hofmiller-Weg 9**
**W-8262 Altötting(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft ein Stabilisatorgemisch, das im wesentlichen aus einer organischen Schwefelverbindung und einer organischen Stickstoffverbindung besteht. Die Erfindung betrifft ferner die Verwendung dieses Stabilisatorgemisches zur Stabilisierung von Oxalkylenglykolalkylethern, insbesondere Oxalkylenglykoldialkylethern.

Oxalkylenglykolalkylether stellen bekanntlich vorteilhafte Lösungs-, Reinigungs- und Verdünnungsmittel dar. In dieser Funktion werden sie beispielsweise zur Herstellung von Lacken, Holzbeizen, Klebstoffen und hydraulischen Flüssigkeiten sowie zur Entfernung von Schwefelwasserstoff und/oder Kohlendioxid aus Natur- oder Synthesegasen und zur Entfernung von Lösemitteln aus Lösemittel enthaltenden Abgasen eingesetzt.

Es ist ferner allgemein bekannt, daß Oxalkylenglykolalkylether unter Einwirkung von oxidierenden Gasen wie Sauerstoff oder Luft Peroxide bilden, aus denen durch weiteren Abbau verfärbend und/oder korrosiv wirkende Zersetzungsprodukte wie Aldehyde, Ester und Säuren entstehen, und daß die Bildung von Peroxiden und deren Abbau durch Wärme gefördert und beschleunigt wird.

Gleichzeitige Anwesenheit von Luft und Wärme tritt insbesondere bei der obenerwähnten Entfernung von Schwefelwasserstoff und/oder Kohlendioxid und bei der obenerwähnten Entfernung von Lösemitteln aus entsprechenden Gasen auf. Die Durchführung dieser Reinigung erfolgt kurz zusammengefaßt in der Weise, daß man die zu reinigenden Gase, wie Koksofengase, Kohlevergasungsgase, Synthesegase und Erdgase sowie die genannten Abgase,durch eine mit vorzugsweise Oxalkylenglykoldialkylethern gefüllte Einrichtung leitet, wobei die genannten sauren Bestandteile Schwefelwasserstoff und Kohlendioxid und die Lösemittel, wie Methylenchlorid, Trichlormethan, Dichlordifluormethan, Dichlortetrafluorethan und dergleichen, absorbiert oder herausgelöst werden. Zur Desorption wird der mit Gasen beladene Oxalkylenglykoldialkylether in der Regel bei einer Temperatur bis zu etwa 150 °C gestript; zur Entfernung oder Rückgewinnung der Lösemittel wird ebenfalls gestript beziehungsweise destilliert. Bei dieser Behandlung der beladenen Oxalkylenglykoldialkylether ist die Gegenwart von Luft selbst bei Stippung oder Destillation mit einem Inertgas in der Regel nicht zu vermeiden. Hier wirken also hohe Temperatur und Luft (als oxidierendes Medium) gleichzeitig auf die als Absorptionsmittel eingesetzten Oxalkylenglykoldialkylether ein. Dazu kommt noch, daß häufig katalytisch wirkende Substanzen, wie zum Beispiel Schwefelsäure,anwesend sind, was den obenerwähnten Abbau der in Rede stehenden Ether unter Umständen noch weiter fördert.

Es sind bereits zahlreiche als Antioxidantien bezeichnete Verbindungen bekannt, deren Einsatz überall dort empfohlen wird, wo mit Peroxidbildung oder mit Oxidationsreaktionen anderer Art zu rechnen ist. Um einige Vertreter aus der überaus großen Zahl der als Antioxidantien beschriebenen Verbindungen zu nennen, seien beispielsweise genannt alkyl- oder aryl-substituierte Phenylendiamine, alkyl-substituierte Phenole, alkyl-substituierte Bisphenole, wie Methylen- oder Thio-bisphenole, Alkyl- oder Arylphosphite und Thioester. Es hat sich gezeigt, daß mit diesen Verbindungen die in Rede stehende Stabilisierung von Oxalkylenglykolalkylethern nicht in zufriedenstellender Weise erreicht wird, selbst wenn sie in einer relativ großen Menge eingesetzt werden.

Es sind auch schon Gemische von zwei oder mehreren Antioxidantien als Stabilisator empfohlen worden, weil dabei ein synergistischer Effekt erwartet wird. So ist in der US-Patentschrift 4 604 417 ein Stabilisatorgemisch aus mindestens einer organischen Schwefelverbindung und mindestens einer organischen Stickstoffverbindung beschrieben. Bei der Schwefelverbindung handelt es sich um spezielle Thioester; bei der Stickstoffverbindung, die durch Phenolderivate ersetzt werden kann, handelt es sich um Napthylamine, Diphenylamine, Chinoline und/oder para-Phenylendiamine, beispielsweise um ein Di-aryl-para-phenylendiamin. In der genannten US-Patentschrift wird dieses Stabilisatorgemisch zur Stabilisierung von vulkanisierten oder unvulkanisierten Polymeren empfohlen.

Die Aufgabe der Erfindung liegt nun darin, eine Stabilisatormischung für Oxalkylenglykolalkylether, insbesondere Oxalkylenglykoldialkylether zu finden, die diese Ether auch in Fällen höherer Temperatur und gleichzeitiger Anwesenheit von oxidierenden Gasen insbesondere gegenüber Peroxidbildung und weiteren Abbaureaktionen langanhaltend stabilisiert.

Das erfindungsgemäße Stabilisatorgemisch, bestehend im wesentlichen aus einer organischen Schwefelverbindung und einer organischen Stickstoffverbindung, ist dadurch gekennzeichnet, daß es im wesentlichen aus

a) 10 bis 90 Gew.-%, vorzugsweise 50 bis 80 Gew.-%, von mindestens einer Verbindung der nachstehenden Formel I,

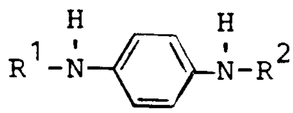

worin $R^1$ und $R^2$, die gleich oder verschieden sein können, bedeuten: einen Alkylrest mit 1 bis 12 Kohlenstoffatomen, vorzugsweise 1 bis 10 Kohlenstoffatomen (der geradkettig oder verzweigt sein kann); einen Cycloalkylrest mit 5 bis 8 Kohlenstoffatomen, vorzugsweise Cyclopentyl oder Cyclohexyl; oder einen Phenyl- oder Naphthylrest, der ein- oder mehrfach mit (geradkettigen oder verzweigten) Alkylresten mit 1 bis 10 Kohlenstoffatomen, vorzugsweise 1 bis 4 Kohlenstoffatomen, substituiert sein kann, wobei ein unsubstituierter oder ein einfach alkyl-substituierter Phenylrest oder Naphthylrest bevorzugt ist; von den beiden Resten ist der Phenylrest bevorzugt;
und
b) 10 bis 90 Gew.-%, vorzugsweise 20 bis 50 Gew.-%, von mindestens einer Verbindung der nachstehenden Formel II

$R^3$-S-$R^4$

worin $R^3$ für einen Alkylrest mit 6 bis 18 Kohlenstoffatomen, vorzugsweise 8 bis 14 Kohlenstoffatomen (der geradkettig oder verzweigt sein kann); einen Rest der Formel $-CH_2(CH_2)_m-CO-OR^5$, worin $R^5$ ein (geradkettiger oder verzweigter) Alkylrest mit 1 bis 18 Kohlenstoffatomen, vorzugsweise 1 bis 12 Kohlenstoffatomen, und m 0, 1 oder 2 ist; oder für einen Phenyl- oder para-Hydroxyphenylrest, der ein- oder mehrfach mit (geradkettigen oder verzweigten) Alkylresten mit 1 bis 10 Kohlenstoffatomen, vorzugsweise 1 bis 4 Kohlenstoffatomen, substituiert ist, wobei ein zweifach alkylsubstitutierter Phenylrest oder Hydroxyphenylrest bevorzugt ist; und $R^4$ für Wasserstoff oder eine der Bedeutungen von $R^3$ steht, wobei $R^3$ und $R^4$ gleich oder verschieden sein können;

besteht, Gewichtsprozente jeweils bezogen auf das Gewicht des Stabilisatorgemisches.

Das erfindungsgemäße Stabilisatorgemisch stellt einen unerwartet wirksamen Stabilisator für die in Rede stehenden Ether, insbesondere für Oxalkylenglykoldialkylether dar. Auch bei hoher Temperatur und gleichzeitiger Anwesenheit von Luft wird eine gegen Peroxidbildung langanhaltende Stabilisierung erreicht. Dieses Ergebnis ist überraschend, nachdem die Wirksamkeit der beiden Verbindungen der Formeln I und II, jeweils allein eingesetzt, weit geringer ist als die Wirksamkeit des angegebenen Verbindungsgemisches. Es liegt offensichtlich ein Synergismus vor, der in diesem Ausmaß nicht zu erwarten war.

Die Herstellung des erfindungsgemäßen Stabilisatorgemisches erfolgt einfach durch Zusammenmischen der Komponenten a) und b), vorzugsweise bei Raumtemperatur und unter Rühren. Die dem erfindungsgemäßen Stabilisatorgemisch zugrundeliegenden Komponenten a) und b) sind bekannt und im Handel erhältlich.

Das erfindungsgemäße Stabilisatorgemisch wird zur Stabilisierung von Oxalkylenglykolalkylethern, vorzugsweise Oxalkylenglykoldialkylethern verwendet (Polyethylen- und/oder Polypropylenglykolether). Bei diesen Ethern handelt es sich vorzugsweise um solche der nachstehenden Formel

$R^6O(CH_2CHR^7O)_nR^8$ ,

worin $R^6$ für einen Alkylrest mit 1 bis 4 Kohlenstoffatomen, $R^7$ für H oder $CH_3$, n für eine Zahl von 1 bis 50, vorzugsweise 1 bis 20 und insbesondere 1 bis 10, und $R^8$ für H oder eine der Bedeutungen von $R^6$ steht, wobei die Dialkylether bevorzugt sind. Bezüglich der Oxethylen- und Oxpropylenreste sind die Oxethylenreste enthaltenden Ether bevorzugt. Für den eingangs erwähnten Einsatz von Oxalkylenglykoldialkylethern zur Entfernung von $H_2S$ und $CO_2$ oder Lösemittel aus entsprechenden Gasen wird nach dem derzeitigen Stand der Technik vor allem eine Mischung von Dialkylethern von Polyethylenglykolen mit 2 bis 10 Ethylenoxid-Einheiten eingesetzt, wobei die beiden Alkylreste zwei Methylreste, ein Methyl- und ein Isopropylrest oder ein $C_1$- bis $C_4$- Alkylrest und ein tertiärer Butylrest sind.

Bei der erfindungsgemäßen Verwendung wird der beschriebene Stabilisator den zu stabilisierenden Oxalkylenglykolalkylethern in einer wirksamen Menge zugesetzt. Dieses Zusetzen oder Einmischen wird in der Regel bei Raumtemperatur und unter Rühren durchgeführt. Die wirksame Menge an erfindungsgemäßem Stabilisatorgemisch kann in weiten Grenzen variieren. Sie hängt insbesondere von der Temperatur des zu stabilisierenden Ethers oder Ethergemisches und von der Anwesenheit eines oxidierenden Gases wie Luft ab und liegt im allgemeinen im Bereich von 0,01 bis 5 Gew.-%, vorzugsweise 0,1 bis 2 Gew.-%,

Gewichtsprozente bezogen auf das Gewicht des zu stabilisierenden Ethers oder Ethergemisches. Bei der erfindungsgemäßen Verwendung wird in der Regel das Gemisch aus den beiden Stabilisator-Komponenten a) und b) eingesetzt. Die Stabilisierung kann aber auch in der Weise durchgeführt werden, daß die beiden Stabilisatorkomponenten getrennt voneinander eingesetzt werden.

Die Erfindung wird nun an Beispielen noch näher erläutert. In den Beispielen sind als Verbindungen der Formeln I und II die nachstehend angegebenen Vertreter eingesetzt worden; bei diesen Formeln mit den Nummern (1) bis (7) handelt es sich um Vertreter der allgemeinen Formel I, das ist die Stabilisatorkomponente a), und bei den nachstehenden Formeln mit den Nummern (8) bis (15) um Vertreter der allgemeinen Formel II, das ist die Stabilisatorkomponente b):

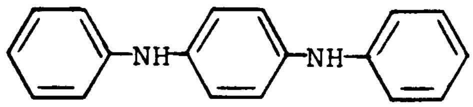

(1)

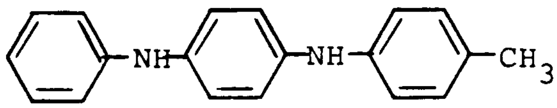

(2)

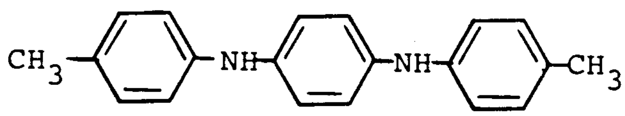

(3)

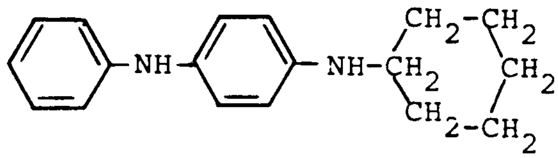

(4)

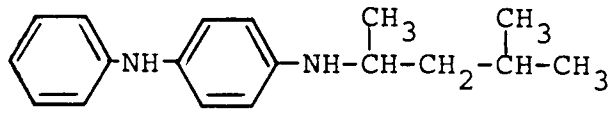

(5)

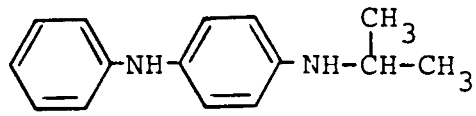

(6)

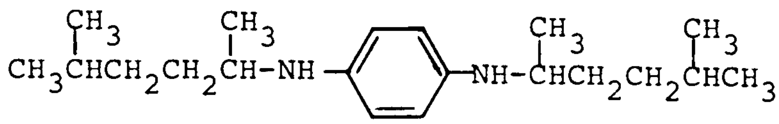

(7)

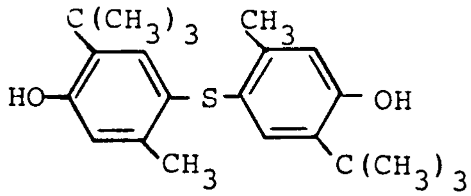

(8)

$C_8H_{17}-S-C_8H_{17}$ (9) $C_{12}H_{25}-S-C_{12}H_{25}$ (10)

$C_{12}H_{25}-S-H$ (11) iso-$C_8H_{17}-O-\overset{\overset{O}{\|}}{C}-CH_2-S-H$ (12)

$$C_{12}H_{25}-O-\overset{\overset{O}{\|}}{C}-CH_2CH_2-S-CH_2-CH_2-\overset{\overset{O}{\|}}{C}-O-C_{12}H_{25} \quad (13)$$

$$CH_3-O-\overset{\overset{O}{\|}}{C}-CH_2CH_2-S-CH_2-CH_2-\overset{\overset{O}{\|}}{C}-O-CH_3 \quad (14)$$

$$C_{18}H_{27}-O-\overset{\overset{O}{\|}}{C}-CH_2CH_2-S-CH_2CH_2-\overset{\overset{O}{\|}}{C}-O-C_{18}H_{27} \quad (15)$$

Der Test der erfindungsgemäßen Stabilisatormischung ist durchgeführt worden mit Polyethylenglykoldimethylether der Molmasse 600 und mit einer Polyethylenglykoldimethylether-Mischung entsprechend der Formel $CH_3O(CH_2CH_2O)_xCH_3$, x = 3 bis 9, wobei der Dimethylether mit x = 4 der Hauptbestandteil der Mischung ist. Es sind zwei Testmethoden angewandt worden; die eine Testmethode wird als "Test mit einer Thermowaage" oder als "thermogravimetrischer Test" und die andere als "Test unter Luftbegasung" bezeichnet. Beide Methoden werden nachstehend im einzelnen beschrieben.

Für jeden Test mit der Thermowaage wird eine bestimmte Menge der beiden Stabilisatorkomponenten a) und b) in dem für den Test eingesetzten Ether, der im vorliegenden Fall ein Polyethylenglykoldimethylether der Molmasse 600 ist, gelöst (die Menge der beiden Stabilisatorkomponenten wird so gewählt, daß sie im Bereich der beschriebenen Zusammensetzung des erfindungsgemäßen Stabilisatorgemisches liegt, und es wird soviel Glykolether eingesetzt, daß in der Lösung die beschriebene Menge an erfindungsgemäßem Stabilisatorgemisch vorliegt). Von der bereiteten Lösung des erfindungsgemäßen Stabilisatorgemisches im Glykolether werden für jeden Test 80 mg mit Hilfe einer Injektionsspritze in ein schalenförmiges Glasgefäßchen gegeben. Das Glasgefäßchen mit der Testprobe wird an der Waage (Thermowaage) aufgehängt und in einen auf 180 °C gehaltenen Ofen gebracht. Im Ofen liegt die übliche Luftatmosphäre vor (er ist also nach außen nicht verschlossen und er wird auch nicht mit einem Inertgas gespült). An der Thermowaage werden das Anfangsgewicht der Testprobe und das nach der gewählten Testzeit vorliegende Endgewicht der Testprobe festgehalten (in der Regel ist die Thermowaage an einen Schreiber angeschlossen, so daß

das Gewicht der Testprobe, bezogen auf die Testzeit, kontinuierlich aufgezeichnet wird). Aus der Differenz des Anfangsgewichtes und des Endgewichtes ergibt sich die Gewichtsabnahme der Testprobe während der Testzeit (aufgrund von Zersetzungsreaktionen). Es ist jene Testzeit in Minuten festgehalten worden, nach der die Gewichtsabnahme 25 %, bezogen auf die eingesetzten 80 mg Testprobe, betragen hat. Diese Zeit stellt eine Maßzahl für die Wirkung des eingesetzten Stabilisators dar (je länger diese Zeit ist, um so wirksamer ist der eingesetzte Stabilisator).

Der Test unter Luftbegasung ist mit Hilfe eines zylinderförmigen Glasgefäßes (Länge 30 cm, Durchmesser 5 cm) mit einer Glasfritte als Boden durchgeführt worden. Für jeden Test wird zunächst eine bestimmte Menge der beiden Stabilisatorkomponenten a) und b) in 350 ml des für den Test eingesetzten Ethers, der im vorliegenden Fall die obengenannte Polyethylenglykoldimethylether-Mischung ist, gelöst (die Menge der beiden Stabilisatorkomponenten wird auch hier so gewählt, daß sie im Bereich der beschriebenen Zusammensetzung des erfindungsgemäßen Stabilisatorgemisches liegt, und daß in der Lösung die beschriebene Menge an erfindungsgemäßem Stabilisatorgemisch vorliegt). Die 350 ml Lösung (Testprobe) wird in das Glasgefäß gegeben und dieses in ein Ölbad gestellt. Das Ölbad und damit auch die Testprobe werden auf 150 °C erhitzt und bei dieser Temperatur gehalten. Nach Erreichen der Temperatur von 150 °C wird ein Luftstrom von 2 l Luft pro Stunde durch die Glasfritte und damit durch die Testprobe hindurchgeleitet. Nach einer Begasungszeit von 150 Stunden wird die Testprobe abgekühlt und auf Zersetzungsreaktionen während der Testzeit untersucht. Dazu wird die Verseifungszahl der behandelten Testprobe ermittelt. Sie stellt eine Maßzahl für die Wirkung des eingesetzten Stabilisators dar (je kleiner die Verseifungszahl ist, um so wirksamer ist der eingesetzte Stabilisator).

Beispiele 1, 4 bis 9, 13, 15, 16 und 18

Diese erfindungsgemäßen Beispiele sind nach dem Test mit der Thermowaage durchgeführt worden. In der nachstehenden Tabelle sind angegeben die eingesetzten Vertreter der Stabilisatorkomponenten a) und b) und ihre Menge im Stabilisatorgemisch in Gewichtsprozent (Gewichtsprozente bezogen auf das Gewicht des Stabilisatorgemisches), die Menge an Stabilisatorgemisch in der Testprobe in Gewichtsprozent (Gewichtsprozente bezogen auf das Gewicht der Testprobe) und das Ergebnis des Testes, das ist die Zeit in Minuten, nach der eine 25 gew.-%ige Abnahme der Testprobe erreicht war. Die Testproben für den Test mit der Thermowaage werden als Testproben 1 bezeichnet.

Beispiele 1 bis 5, 10, 11, 12, 14 und 17

Diese erfindungsgemäßen Beispiele sind nach dem Test unter Luftbegasung durchgeführt worden. In der nachstehenden Tabelle sind die dem Test mit der Thermowaage analogen Angaben zusammengefaßt, wobei das Ergebnis des Testes die Verseifungszahl der behandelten Probe ist. Die Testproben für den Test unter Luftbegasung werden als Testproben 2 bezeichnet.

Die erfindungsgemäßen Beispiele 1, 4 und 5 sind also nach den beiden Testmethoden durchgeführt worden. Die Vertreter mit den Nummern (1), (2) und (3) sind in Form einer im Handel erhältlichen Mischung eingesetzt worden.

Vergleichsbeispiele 19 und 20

Im Vergleichsbeispiel 19 ist ein Vertreter der Komponente a) des erfindungsgemäßen Stabilisatorgemisches allein und im Vergleichsbeispiel 20 ein Vertreter der Komponente b) allein nach dem Test mit der Thermowaage und nach dem Test unter Luftbegasung geprüft worden. Die zu den erfindungsgemäßen Beispielen analogen Angaben und Ergebnisse sind in der nachstehenden Tabelle zusammengefaßt.

Blindwerte

Die Blindwerte sind das Ergebnis der Tests der vollständig unstabilisierten Dimethylether, das sind der obengenannte Polyethylenglykoldimethylether der Molmasse 600 (in der nachstehenden Tabelle als "Ether 1" bezeichnet) und die obengenannte Polyethylenglyokoldimethylether-Mischung (in der nachstehenden Tabelle als "Ether 2" bezeichnet). Die beiden Testergebnisse sind in der Tabelle angeführt.

Die Ergebnisse der Tests zeigen, daß die erfindungsgemäße Stabilisatormischung einen unerwartet hohen Synergismus aufweist und damit eine überaus wirksame Stabilisierung ergibt.

T A B E L L E

| Beispiel Nr. | Stabilisatorgemisch: Vertreter für die Komponente a) Nr. | Gew.-% | Vertreter für die Komponente b) Nr. | Gew.-% | Menge an Stabilisatorgemisch in der Testprobe 1 Gew.-% | Menge an Stabilisatorgemisch in der Testprobe 2 Gew.-% | Ergebnis: Zeit nach einer 25 %igen Gewichtsabnahme der Testprobe in Minuten | Verseifungszahl der Testprobe nach dem Test |
|---|---|---|---|---|---|---|---|---|
| 1 | 5 | 50 | 8 | 50 | 1 | 0,5 | 8060 | 9 |
| 2 | 4 | 60 | 8 | 40 | - | 0,5 | - | 17 |
| 3 | 5 | 50 | 11 | 50 | - | 0,5 | - | 20 |
| 4 | 1 bis 3 | 50 | 8 | 50 | 1 | 0,5 | 5965 | 38 |
| 5 | 1 bis 3 | 80 | 8 | 20 | 1 | 0,5 | 5620 | 35 |
| 6 | 1 bis 3 | 60 | 8 | 40 | 1 | - | 6825 | - |
| 7 | 1 bis 3 | 70 | 8 | 30 | 1 | - | 5200 | - |
| 8 | 1 bis 3 | 90 | 8 | 10 | 1 | - | 1580 | - |
| 9 | 1 bis 3 | 20 | 8 | 80 | 1 | - | 1920 | - |
| 10 | 1 bis 3 | 50 | 14 | 50 | - | 0,5 | - | 38 |
| 11 | 1 bis 3 | 50 | 13 | 50 | - | 0,5 | - | 38 |
| 12 | 1 bis 3 | 60 | 9 | 40 | - | 0,5 | - | 9 |
| 13 | 5 | 50 | 9 | 50 | 1 | - | 7000 | - |
| 14 | 5 | 50 | 10 | 50 | - | 0,5 | - | 36 |
| 15 | 6 | 50 | 8 | 50 | 1 | - | 3500 | - |
| 16 | 7 | 50 | 15 | 50 | 1 | - | 1550 | - |
| 17 | 1 bis 3 | 50 | 12 | 50 | - | 0,5 | - | 30 |
| 18 | 5 | 50 | 9 | 50 | 0,5 | - | 2225 | - |

TABELLE (fortgesetzt)

| Beispiel Nr. | Stabilisatorgemisch: Vertreter für die Komponente a) Nr. | Gew.-% | Vertreter für die Komponente b) Nr. | Gew.-% | Menge an Stabilisatorgemisch in der Testprobe 1 Gew.-% | Menge an Stabilisatorgemisch in der Testprobe 2 Gew.-% | Ergebnis: Zeit nach einer 25 %igen Gewichtsabnahme der Testprobe in Minuten | Ergebnis: Verseifungszahl der Testprobe nach dem Test |
|---|---|---|---|---|---|---|---|---|
| Vergleichsbeispiele | | | | | | | | |
| 19 | 1 bis 3 | 100 | - | - | 1 | 0,5 | 939 | 40 |
| 20 | - | - | 8 | 100 | 1 | 0,5 | 710 | 42 |
| Blindwerte | | | | | | | | |
| Ether 1 | - | - | - | - | - | - | - | 145 |
| Ether 2 | - | - | - | - | - | - | 162 | - |

## Patentansprüche

1. Stabilisatorgemisch, bestehend im wesentlichen aus einer organischen Schwefelverbindung und einer organischen Stickstoffverbindung, dadurch gekennzeichnet, daß es im wesentlichen aus

a) 10 bis 90 Gew.-% von mindestens einer Verbindung der nachstehenden Formel I,

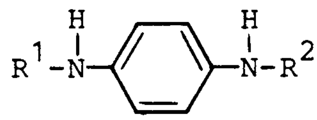

worin $R^1$ und $R^2$, die gleich oder verschieden sein können, bedeuten: einen Alkylrest mit 1 bis 12 Kohlenstoffatomen, einen Cycloalkylrest mit 5 bis 8 Kohlenstoffatomen oder einen Phenyl- oder Naphthylrest, der ein- oder mehrfach mit Alkylresten mit 1 bis 10 Kohlenstoffatomen substituiert sein kann,

und

b) 10 bis 90 Gew.-% von mindestens einer Verbindung der nachstehenden Formel II,

$R^3$-S-$R^4$

worin $R^3$ für einen Alkylrest mit 6 bis 18 Kohlenstoffatomen, einen Rest der Formel $-CH_2(CH_2)_m-CO-OR^5$, worin $R^5$ ein Alkylrest mit 1 bis 18 Kohlenstoffatomen und m 0, 1 oder 2 ist, oder für einen Phenyl- oder para-Hydroxyphenylrest, der ein- oder mehrfach mit Alkylresten mit 1 bis 10 Kohlenstoffatomen substituiert ist, und $R^4$ für Wasserstoff oder eine der Bedeutungen von $R^3$ steht, wobei $R^3$ und $R^4$ gleich oder verschieden sein können,

besteht, Gewichtsprozente jeweils bezogen auf das Gewicht des Stabilisatorgemisches.

**2.** Stabilisatorgemisch nach Anspruch 1, dadurch gekennzeichnet, daß in der Formel I $R^1$ und $R^2$ einen Alkylrest mit 1 bis 10 Kohlenstoffatomen, den Cyclopentyl- oder den Cyclohexylrest, oder einen Phenyl- oder Naphthylrest, der ein- oder mehrfach mit Alkylresten mit 1 bis 4 Kohlenstoffatomen substituiert sein kann, bedeuten, und in der Formel II $R^3$ für einen Alkylrest mit 8 bis 14 Kohlenstoffatomen, einen Rest der Formel $-CH_2(CH_2)_m-CO-OR^5$, worin $R^5$ ein Alkylrest mit 1 bis 12 Kohlenstoffatomen und m 0, 1 oder 2 ist, oder für einen Phenyl- oder para-Hydroxyphenylrest, der ein- oder mehrfach mit Alkylresten mit 1 bis 4 Kohlenstoffatomen substituiert ist, und $R^4$ für Wasserstoff oder eine der Bedeutungen von $R^3$ steht.

**3.** Stabilisatorgemisch nach Anspruch 1, dadurch gekennzeichnet, daß in der Formel I $R^1$ und $R^2$ einen Alkylrest mit 1 bis 10 Kohlenstoffatomen, den Cyclopentyl- oder den Cyclohexylrest, oder einen unsubstituierten oder einen einfach $C_1$- bis $C_4$-Alkyl-substituierten Phenyl- oder Naphthylrest bedeuten, und in der Formel II $R^3$ für einen Alkylrest mit 8 bis 14 Kohlenstoffatomen, einen Rest der Formel $-CH_2-(CH_2)_m-CO-OR^5$, worin $R^5$ ein Alkylrest mit 1 bis 12 Kohlenstoffatomen und m 0, 1 oder 2 ist, oder für einen Phenyl- oder para-Hydroxyphenylrest, der zweifach $C_1$- bis $C_4$-Alkyl-substituiert ist, und $R^4$ für Wasserstoff oder eine der Bedeutungen von $R^3$ steht.

**4.** Stabilisatorgemisch nach Anspruch 1, dadurch gekennzeichnet, daß in der Formel I $R^1$ und $R^2$ einen Alkylrest mit 1 bis 10 Kohlenstoffatomen, den Cyclopentyl- oder den Cyclohexylrest, oder einen unsubstituierten oder einen einfach $C_1$- bis $C_4$-Alkyl-substituierten Phenylrest bedeuten, und in der Formel II $R^3$ für einen Alkylrest mit 8 bis 14 Kohlenstoffatomen, einen Rest der Formel $-CH_2(CH_2)_m-CO-OR^5$, worin $R^5$ ein Alkylrest mit 1 bis 12 Kohlenstoffatomen und m 0, 1 oder 2 ist, oder für einen para-Hydroxyphenylrest, der zweifach $C_1$- bis $C_4$-Alkyl-substituiert ist, und $R^4$ für Wasserstoff oder eine der Bedeutungen von $R^3$ steht.

**5.** Stabilisatorgemisch nach einem der Ansprüche 1 bis 4, dadurch gekennzeichnet, daß es die Komponente a) in einer Menge von 50 bis 80 Gew.-% und die Komponente b) in einer Menge von 20 bis 50 Gew.-% enthält.

**6.** Verwendung des Stabilisatorgemisches nach den Ansprüchen 1 bis 5 zur Stabilisierung von Oxalkylenglykolalkylethern.

**7.** Verwendung des Stabilisatorgemisches nach den Ansprüchen 1 bis 5 zur Stabilisierung von Oxalkylenglykoldialkylethern.

## Claims

1. A stabilizer composition consisting essentially of an organic sulfur containing compound and of an organic nitrogen containing compound, characterized in that said composition consists essentially of
a) 10 to 90 % by weight of at least one compound of the following formula I

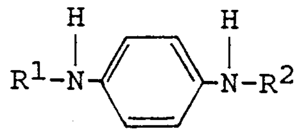

in which $R^1$ and $R^2$, which are identical or different, denote an alkyl radical having 1 to 12 carbon atoms, a cycloalkyl radical having 5 to 8 carbon atoms, or a phenyl or naphthyl radical which can be singly or manifoldy substituted with alkyl radicals having 1 to 10 carbon atoms, and
b) 10 to 90 % by weight of at least one compound of the following formula II

$R^3$-S-$R^4$

in which $R^3$ denotes an alkyl radical having 6 to 18 carbon atoms, a radical of the formula $-CH_2-(CH_2)_m-CO-OR^5$, wherein $R^5$ is an alkyl radical having 1 to 18 carbon atoms and m is 0, 1, or 2, or a phenyl or para-hydroxyphenyl radical which is singly or manifoldy substituted with alkyl radicals having 1 to 10 carbon atoms, and $R^4$ denotes hydrogen or one of the meanings of $R^3$, $R^3$ and $R^4$ being identical or different, said percentages by weight being relative to the weight of the stabilizer composition.

2. A stabilizer composition as claimed in claim 1, wherein in formula I $R^1$ and $R^2$ denote an alkyl radical having 1 to 10 carbon atoms, the cyclopentyl or the cyclohexyl radical, or a phenyl or naphthyl radical which can be singly or manifoldy substituted with alkyl radicals having 1 to 4 carbon atoms, and in formula II $R^3$ denotes an alkyl radical having 8 to 14 carbon atoms, a radical of the formula $-CH_2(CH_2)_m-CO-OR^5$, wherein $R^5$ is an alkyl radical having 1 to 12 carbon atoms and m is 0, 1, or 2, or a phenyl or para-hydroxyphenyl radical which is singly or manifoldy substituted with alkyl radicals having 1 to 4 carbon atoms, and $R^4$ denotes hydrogen or one of the meanings of $R^3$.

3. A stabilizer composition as claimed in claim 1, wherein in formula I $R^1$ and $R^2$ denote an alkyl radical having 1 to 10 carbon atoms, the cyclopentyl or the cyclohexyl radical, or an unsubstituted or a singly $C_1$ to $C_4$-alkyl substituted phenyl or naphthyl radical, and in formula II $R^3$ denotes an alkyl radical having 8 to 14 carbon atoms, a radical of the formula $-CH_2(CH_2)_m-CO-OR^5$, wherein $R^5$ is an alkyl radical having 1 to 12 carbon atoms and m is 0, 1, or 2, or a phenyl or para-hydroxyphenyl radical which is twofoldy $C_1$ to $C_4$-alkyl substituted, and $R^4$ denotes hydrogen or one of the meanings of $R^3$.

4. A stabilizer composition as claimed in claim 1, wherein in formula I $R^1$ and $R^2$ denote an alkyl radical having 1 to 10 carbon atoms, the cyclopentyl or the cyclohexyl radical, or an unsubstituted or a singly $C_1$ to $C_4$-alkyl substituted phenyl radical, and in formula II $R^3$ denotes an alkyl radical having 8 to 14 carbon atoms, a radical of the formula $-CH_2(CH_2)_m-CO-OR^5$, wherein $R^5$ is an alkyl radical having 1 to 12 carbon atoms and m is 0, 1, or 2, or a para-hydroxyphenyl radical which is twofoldy $C_1$ to $C_4$-alkyl substituted, and $R^4$ denotes hydrogen or one of the meanings of $R^3$.

5. A stabilizer composition as claimed in claims 1 to 4, wherein the composition contains the component a) in an amount of 50 to 80 % by weight and the component b) in an amount of 20 to 50 % by weight.

6. The use of the stabilizer composition of claims 1 to 5 for the stabilization of oxalkylene glycol ethers.

7. The use of the stabilizer composition of claims 1 to 5 for the stabilization of oxalkylene glycol diethers.

## Revendications

**1.** Mélange stabilisant, consistant essentiellement en un composé organique du soufre et un composé organique de l'azote, mélange caractérisé en ce qu'il consiste essentiellement en :

a) 10 à 90 % en poids d'au moins un composé répondant à la formule I suivante,

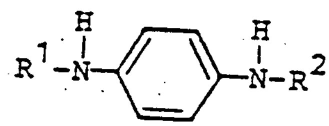

(dans laquelle $R^1$ et $R^2$, qui peuvent être identiques ou différents, représentent chacun un reste alkyle ayant 1 à 12 atomes de carbone, un reste cycloalkyle ayant 5 à 8 atomes de carbone ou un reste phényle ou naphtyle, qui peut être substitué une ou plusieurs fois par des restes alkyles ayant chacun 1 à 10 atomes de carbone), et

b) 10 à 90 % en poids d'au moins un composé répondant à la formule II ci-après,

$R^3$-S-$R^4$

(dans laquelle $R^3$ représente un reste alkyle ayant 6 à 10 atomes de carbone, un reste de formule $-CH_2(CH_2)_m-CO-OR^5$, dans laquelle $R^5$ représente un reste alkyle ayant 1 à 18 atomes de carbone et m vaut 0, 1 ou 2, ou un reste phényle ou para-hydroxyphényle qui est substitué une ou plusieurs fois par des restes alkyles ayant chacun 1 à 10 atomes de carbone, et $R^4$ représente un atome d'hydrogène ou a l'un des sens de $R^3$, les symboles $R^3$ et $R^4$ pouvant être identiques ou différents,

les pourcentages en poids étant toujours rapportés au poids du mélange stabilisant.

**2.** Mélange stabilisant selon la revendication 1, caractérisé en ce que, dans la formule I, $R^1$ et $R^2$ représentent chacun un reste alkyle ayant 1 à 10 atomes de carbone, le reste cyclopentyle ou le reste cyclohexyle, ou un reste phényle ou naphtyle, qui peut être substitué une ou plusieurs fois par des restes alkyles ayant 1 à 4 atomes de carbone ; et, dans la formule II, $R^3$ représente un reste alkyle ayant 8 à 14 atomes de carbone, un reste de formule $-CH_2(CH_2)_m-CO-OR^5$, dans laquelle $R^5$ représente un reste alkyle ayant 1 à 12 atomes de carbone, et m vaut 0, 1 ou 2, ou $R^3$ représente un reste phényle ou para-hydroxyphényle, qui est substitué une ou plusieurs fois par des restes alkyles ayant chacun 1 à 4 atomes de carbone, et $R^4$ représente un atome d'hydrogène ou a l'un des sens de $R^3$.

**3.** Mélange stabilisant selon la revendication 1, caractérisé en ce que, dans la formule I, $R^1$ et $R^2$ représentent chacun un reste alkyle ayant 1 à 10 atomes de carbone, le reste cyclopentyle ou le reste cyclohexyle, ou un reste phényle ou naphtyle non substitué ou substitué par un groupe alkyle en $C_1$ à $C_4$, et, dans la formule II, $R^3$ représente un reste alkyle comportant 8 à 14 atomes de carbone, un reste de formule $-CH_2(CH_2)_m-CO-OR^5$, dans laquelle $R^5$ représente un reste alkyle ayant 1 à 12 atomes de carbone et m vaut 0, 1 ou 2, ou un reste phényle ou para-hydroxyphényle, qui est substitué deux fois par un reste alkyle en $C_1$ à $C_4$, et $R^4$ représente un atome d'hydrogène ou a l'un des sens de $R^3$.

**4.** Mélange stabilisant selon la revendication 1, caractérisé en ce que, dans la formule I, $R^1$ et $R^2$ représentent chacun un reste alkyle ayant 1 à 10 atomes de carbone, le reste cyclopentyle ou le reste cyclohexyle, ou un reste phényle non substitué ou substitué par un groupe alkyle en $C_1$ à $C_4$, et, dans la formule II, $R^3$ représente un reste alkyle ayant 8 à 14 atomes de carbone, un reste de formule $-CH_2-(CH_2)_m-CO-OR^5$, dans laquelle $R^5$ représente un reste alkyle ayant 1 à 12 atomes de carbone et m vaut 0, 1 ou 2, ou un reste para-hydroxyphényle qui porte deux substituants alkyles en $C_1$ à $C_4$, et $R^4$ représente un atome d'hydrogène ou a l'un des sens de $R^3$.

**5.** Mélange stabilisant selon l'une des revendications 1 à 4, caractérisé en ce qu'il contient le composant (a) présent en une quantité de 50 à 80 % en poids et le composant (b) présent en une quantité de 20 à 50 % en poids.

**6.** Utilisation du mélange stabilisant selon les revendications 1 à 5 pour stabiliser des éthers alkyliques d'oxyalkylène-glycols.

7. Utilisation du mélange stabilisant selon les revendications 1 à 5 pour stabiliser des éthers dialkyliques d'oxyalkylène-glycols.